# EUROPEAN PATENT SPECIFICATION

(11) **EP 2 273 004 B1**
(45) Date of publication and mention of the grant of the patent: **26.12.2012**
(21) Application number: 09165034.1
(22) Date of filing: 09.07.2009
(51) Int. Cl.: D06F 43/06

(54) **Cleaning system**
Reinigungssystem
Système de nettoyage

(43) Date of publication of application: 12.01.2011
(73) Proprietor: Elozo Oy, 66440 Tervajoki (FI)
(72) Inventor: Pirhonen, Mikko, 13720 Parola (FI); Parkkali, Markus, 66400 Laihia (FI)
(74) Representative: Niemi, Hakan Henrik

(56) References cited:
- WO-A-00/52249
- FR-A- 2 710 350
- US-A- 3 226 842
- US-A1- 2008 159 907

## Description

### Field

The invention relates to a system of cleaning clothes.

### Background

In many places it would be advantageous if items such as textiles, protective clothing, common use clothes and children's toys could be cleaned often, quickly and in an environmentally friendly way from microbes and odour impurities. Above-mentioned items include, for instance, work clothing used in hospitals, pharmacies, food industry and theatres and toys and suppliers used in day care centres.

Water cleaning or chemical dry cleaning cannot be applied to all clothing due to the sensitivity of the clothing, such as theatre dresses. Some footwear cannot be washed either. When used, odours, sweat and bacteria remain in these kind of items. Furthermore, water cleaning is laborious and hazardous to the environment due to the use of water and chemicals.

US 2008/0159907 discloses a method and an apparatus using ozone for disinfecting and/or deodorizing an article.

### Brief description

It is an object of the invention to alleviate the above mentioned disadvantages. This object is achieved with cabinet having the features of claim 1.

Advantageous embodiments are defined in the dependent-claims.

The invention thus provides a way to clean objects from odours and other impurities quickly and in an environmentally friendly manner, and without mechanical strain on the object.

### Drawings

The invention will now be described with reference to the drawings, in which
Figure 1 shows an embodiment of an arrangement;
Figure 2 shows an embodiment of a method.

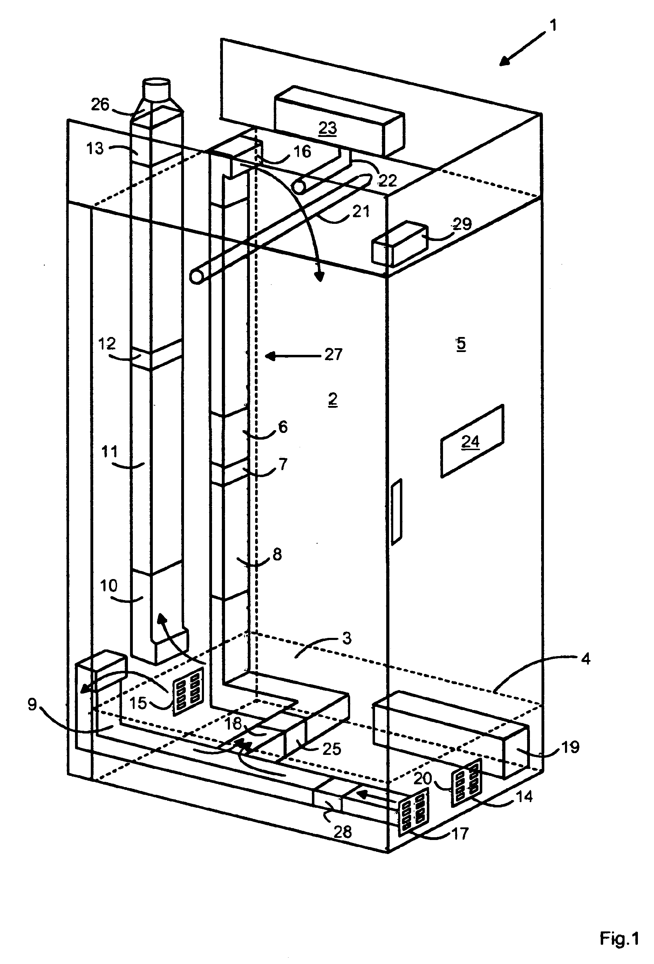

Figure 1 shows an embodiment of an ozone cleaning arrangement. The cabinet 1 is arranged into a form of a cabinet including a cleaning space 2 and a device space 3, which are separated by a wall 4. In the embodiment of Figure 1, the device space 3 is partly located below, behind (when seen from the direction of the door) and above the cleaning space 2. The cleaning space is provided with a door 5 to allow the objects to be cleaned to be placed into the cleaning space and removed from the cleaning space.

The cleaning space defined by the side walls, floor and roof forms a gas tight structure preventing any gas in/outflow to/from the cleaning space. The gas used in the cleaning space includes ozone, which is detrimental to health, and whose distribution to the surroundings of the cabinet 1 is to be prevented. The materials within the cleaning space, and also generally in the cabinet, which come into contact with ozone are preferably ozone-durable and such that they do not dissipate ozone.

A clothes rail 21 may be provided in the cleaning space for hanging the clothes. Alternatively, or in addition, shelves for shoes or other objects may be provided in the cleaning space.

The device space 3 includes a circulation channel 27. The circulation channel 27 may be an elongated tube having an adjustable inlet air opening 17 at the lower part of the tube. The inlet air opening 17 is configured to receive replacement air from the exterior of the cabinet. In the embodiment of Figure 1, cooling air to cool the devices in the device space may be input into the device space 3 via a valve 14. A fan 20 may be provided at the inlet opening to accelerate air flow. Excess air may exit the system via an outlet opening 15. Instead of arranging the openings as shown in Figure 1, it is possible that the inlet opening 17 is facing the interior of the device space thus obtaining replacement air from the exterior of the cabinet via the device space.

In addition to receiving air from the exterior of the cabinet, the circulation channel 27 may also receive air from the cleaning space 2 via a passage 9 connecting the cleaning space 2 and the circulation channel 27.

Within the circulation channel 27, the air first passes through a filter 18 filtering impurities from the input air. The filtered air is passed via a heating element 25, such as a heat resistor, which may warm the input air. The circulation channel 27 also includes a cooling element 8, such as a Peltier element or a heat exchanger, which may cool the input air and, consequently, the ozone to be fed to the cleaning space. To maximize the ozone cleaning properties,

### Description of some embodiments

Triatomic ozone O₃ affects the objects to be cleaned in different ways depending on the microbes or odour molecules on the object. the input air temperature may be adjusted to a predetermined temperature at which the ozone reacts most efficiently with impurities.

A fan 7 accelerates the input air flow 17 to an ozone generator 6. Instead of, or in addition to the fan 7, a fan 28 may be provided at the inlet opening 17 to accelerate air flow to the ozone generation. Accelerated air flow is advantageous in generating an overpressure to the cabinet and thus preventing ozone from escaping the cabinet via the inlet openings.

The operation of the ozone generator 6 may be based on corona discharging or ultraviolet light. The generator 6 generates ozone from oxygen (O₂) by dissipating it first to one-atomic and combining the one-atomic oxygen with two-atomic oxygen to provide tri-atomic oxygen O₃, i.e. ozone. The ozone is passed from the circulation channel 27 to the cleaning space via an opening at the top end 16 of the ozone generation unit.

Instead of a single passage conveying the generated ozone to the cleaning space, a manifold for distributing ozone in the cleaning space may be provided. The manifold may comprise six outlets, for instance. Each of the outlets may be coupled to a corresponding flexible hose, which may be directed to a suitable spot in the cleaning space. For instance, if the object to be cleaned is a fireman's uniform, an end of one of the hoses may be directed to the inside of the uniform. A manifold having several outlets is also advantageous in a situation, where the cleaning space is so big that effective ozone distribution is not achieved with one ozone inlet opening only. By using manifold and connected hoses, the ozone may be effectively conveyed to different positions within the cleaning space to maximize the cleaning effect.

The device space may also include a neutralizing unit. An outlet in the wall between the cleaning space and the device space may be provided and connected to a discharge channel 10 of the neutralizing unit. The ozone and air mixture received from the cleaning space via the outlet opening is fed to a neutralizer 11, which converts the ozone to oxygen. The neutralizer may comprise a container made of stainless steel. The container may be filled with ozone neutralizing grains, which include mangano oxide or absorbent carbon. The gas flow through the container is arranged to be slower than a predetermined threshold setting. The setting may be 0,5 m³ in one minute, for instance to let the neutralizing matter influence the gas to be neutralized in order to sufficiently neutralize the ozone therein. Alternatively, the neutralization may be based on using ultraviolet radiation, heat or chemical neutralization, for instance.

The neutralizing unit may include an exhaust blower 12, such that the air may be removed from the neutralizing unit efficiently. The air may be conveyed out of the arrangement via the end 13 of the neutralizing unit and via an exhaust pipe 26 to a ventilation system of the building containing the arrangement 1, for instance.

The arrangement may further include a water tank 23 and a water spray 22 connected to the water tank. A pump may be provided to pump water from the tank to the water spray such as to moisten the objects in the cleaning space. The cleaning process using ozone is more effective when the surface of the objects is somewhat moist.

The cleaning space is thus closed except for the passage at the top of the cleaning space conveying the ozone and air mixture to the cleaning space, and a first outlet opening conveying air/ozone from the cleaning space back to the ozone generation, and a second outlet opening conveying air/ozone to the ozone neutralizer. The various passages to the cleaning space may be integrally formed, by casting, for instance, to ensure maximal leaktightness. In Figure 1, all the passages to/from the cleaning space have been arranged to the same wall of the cleaning space such as to reduce risk of gas leakage from the cleaning space.

The cabinet of Figure 1 includes a control unit 19 for controlling the operation of the cabinet. The controlling refers to here to starting and/or stopping ozone generation, neutralization, heating, cooling, drying and ventilation, for instance. The controlling of the cabinet may be carried out together with a user interface 24 provided for receiving instructions and selections from the user. The user interface may also present to the user various selection options, measurement information and progress of cleaning programs running in the cabinet.

The control unit provides different entities of the cabinet with operating voltage and control signals. The control unit is also configured to receive measurement information from various sensors of the cabinet. Such sensors/meters (not shown in Figure 1) may include one or more of the following.

A moisture sensor may be provided in the cleaning space 2. If the moisture level in the cleaning space 2 is too low, the control unit may order the pump 23 to pump water to the water spray 22 for spraying water on the objects in the cleaning space. The ozone cleaning is optimal at a certain moisture level, which may be set to the cleaning space by the control unit 19. On the other hand, if the moisture sensor indicates that the objects to be cleaned are too moist, the control unit may control the fans 20 and 12 to intensify air circulation in the cabinet 1 and the heating element 25 to raise the temperature of the input air.

An ozone concentration sensor may be provided in the cleaning space. In an application, the ozone concentration sensor is used to determine when the door of the cabinet may be opened. In an embodiment, after the ozone generation for the cleaning space has been stopped, neutralization still continues. The ozone concentration sensor measures the ozone concentration in the cleaning space and allows the door of the cleaning space to be opened when the ozone concentration falls below a predetermined threshold value.

The ozone concentration sensor is also used to determine an optimal ozone level in view of cleaning. The ozone concentration within the cleaning space may be adjusted in various ways. In an embodiment, the amount of fresh air is adjusted. If the ozone concentration is to be raised, the input of fresh air is diminished either by restricting the air flow in the inlet openings 14, 17 and/or slowing down the fans in order to slow down the gas flow in the system.

In another application, the control unit determines the cleanliness of the clothes in the cleaning space 2. In this application, the control unit stops the ozone generation and neutralization, and closes the cleaning space such that no gas is exchanged between the cleaning space and its surroundings. By measuring the declining speed of the ozone concentration in the cleaning space, the control unit may determine if the clothes are sufficiently clean. That is, if the declining speed of the ozone is higher than a predetermined threshold value, this means that the ozone in the cleaning space still reacts with impurities in the cleaning space and a sufficient cleanliness level has not yet been reached. On the other hand, if the ozone concentration declining speed in the cleaning space undershoots a predetermined threshold value, this means that no or only a minor oxidation reaction happens in the cleaning space, thus indicating that the clothes are sufficiently clean.

Furthermore, the cleaning space may be provided with a temperature sensor. Upon a signal from the temperature sensor, the control unit may adjust the inside temperature depending on the need. For instance, for effective oxidation, the inside temperature of the cleaning space may be set to about 10 degrees Celsius, for instance.

In an embodiment, a security mode is provided when the cleaning process is started, that is the cabinet is switched on. In the security mode, it is required, for instance, that the panel keys of the user interface are pressed in a certain order and the "start" key is pressed for a predetermined time to start the device. In the security mode, unintentional starting of the device becomes practically impossible. The device may be provided with delayed door locking, during which the door of the cabinet can be opened with very little force. During the first three minutes, for instance, the door of the cabinet is kept closed only with magnetic force so that even a small child is capable of opening the door during this security period. Preferably, no ozone is generated during this period. After the security period, the door of the cabinet may be closed with a security lock 29 preventing accidental opening of the door. The security lock may be kept to close the door until the ozone concentration in the cleaning space has declined to a safe level.

In an embodiment, the cleaning space is provided with a motion detector, which is configured to detect and indicate if a living object is within the cleaning space. If a living object is detected, the operation of the cabinet, such as ozone generation, is not started until the door of the cabinet is opened.

The arrangement of Figure 1 is used as follows. First, the items, such as hospital clothes to be cleaned, are placed into the cleaning space 2. The clothes are hung spaciously on the clothes rail 21, and the door 5 is closed. The user inputs the process parameters, such as time and concentration of the cleaning process, on the user interface 24. The total effectiveness may be set by adjusting the product of time T (in minutes) and concentration (ppmv, Parts Per Million by Volume), where the user may select the concentration from a range of 0 to 30 ppmv, for instance.

The use of a concentration 20 ppmv for 15 minutes, for instance, thus gives a total effectiveness CT=300 ppmv minutes.

After the total effectiveness has been set, the cleaning process may be started under the supervision of the control unit 19.

Air is input to the ozone generator 6 via the route 9, 18, 25, 8, and the generated ozone mixed with air is output via the end part 16 to the cleaning space 2. The desired ozone level in the cleaning space may be reached in a few minutes.

When the cleaning program is to be stopped, the ozone concentration within the cleaning space is to be reduced. To achieve this, the ozone generation is stopped, and the air-ozone mixture from the cleaning space is removed by neutralization. At the same time, fresh air is allowed to flow to the cleaning space via the inlet opening 17. When a sufficiently low ozone concentration has been reached in the cleaning space, the door of the cabinet is allowed to be opened.

In an embodiment, a warming and moistening phase is carried out in the cabinet before the ozone cleaning process is started. In the heating and moistening process, bacteria in germ form are converted into biotic form. In this phase, the heating element 25 may warm the air to 37 degrees, for instance, and water mist is sprayed over the clothes in the cleaning space with the spraying element 22.

The embodiment of Figure 1 shows the cleaning and device spaces being separated by a wall. It is to be understood that the construction of the cabinet 1 can be different.

In an embodiment, only a single cleaning space is provided. This one single cleaning space incorporates all units needed for ozone generation and neutralization. In this embodiment, the ozone generator residing within the cleaning space may be configured to have an inlet opening for receiving replacement air from the exterior of the cleaning space. Furthermore, the neutralizing unit residing within the cleaning space is configured to have an outlet opening leading out of the cleaning space. Thus, as also in Figure 1, gas circulation in form of an input flow and an output flow is arranged to the cleaning space.

The ozone generation takes place within the cleaning space such that air from the outside of the cabinet is input to the ozone generator in the cleaning space. For neutralization, however, the reaction product including oxygen and ozone is taken away from the cleaning space and neutralized outside the cleaning space.

In still another embodiment, the ozone generation takes place outside the cleaning space but the neutralization is carried out within the cleaning space such that only oxygen and air are output from the cleaning space.

The cabinet can be used in several modes. In an ozone cleaning mode, ozone generation and neutralization are in use. In the ozone cleaning mode, the temperature provided by the element 25 may be adjusted as desired or the element 25 may be shut off. For the ozone cleaning, it is optimal to have a temperature somewhat lower than the room temperature. Thus, the cooling element 8 may be coupled to cool the ozone and air to 10 degrees Celsius, for instance.

In a second mode, the cabinet may be used as a drying chamber without ozone cleaning. Then, the ozone generator 6 is shut off and only the fans, such as the fan 7, are running to blow air warmed by the heating element 25 to the cleaning space.

In a third mode, the cabinet is used in ventilation/airing of the objects in the cleaning space. In this mode, the aim is mainly to provide ventilation, not drying, and thus the temperature of the air to be conveyed to the cleaning space may be lower than in the second mode.

The different operation modes may be combined. As an example, the user may wish to run a cleaning program for clothes that have been water-washed. The user may want to run ozone cleaning and drying for the clothes. The cabinet may measure the humidity in the cabinet, and upon noticing that the humidity is high, starts drying of the clothes first. When the humidity in the cabinet has declined to a predetermined level, that is, the clothes are still somewhat moist, the ozone generation is started. After the ozone cleaning phase, drying of the clothes may be continued until the clothes are dry. The various operation modes may be combined in many alternative ways, of which the above is only one example.

The various functionalities in the control unit 19 may be implemented by means of software in a computer program loadable to a processor for carrying out the functionality when run on the processor.

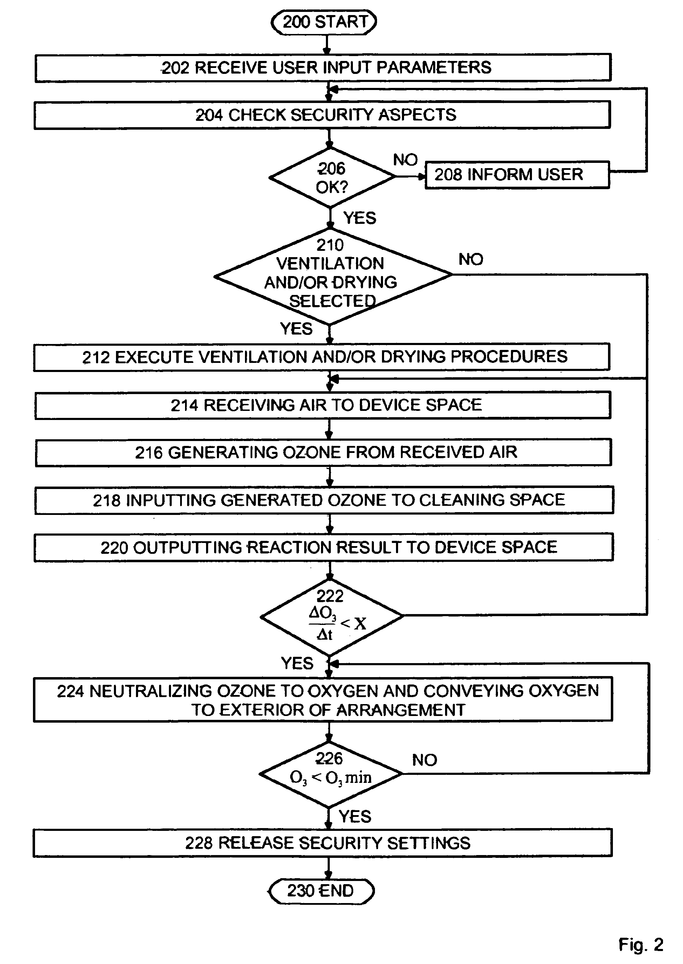

Figure 2 shows an embodiment of a method. The method is carried out in a cabinet suitable for ozone cleaning. The cleaning space of the cabinet is for receiving the items to be cleaned, which may be clothes, shoes or children's toys, for instance. A device space is separated from the cleaning space by a wall but the two spaces are operationally coupled as explained in the following.

In 202, user input parameters are received via a user interface. The user may select one or more cleaning processes including ventilation, drying and ozone cleaning. The user may also provide input parameters such as time and efficiency of the various processes.

In 204/206, security aspects are checked. Checking of security aspects may include checking that no movement has been detected within the cleaning space during a security period of three minutes. If the security aspects are not complied with, the cabinet may inform 208 the user of the reason of the non-compliance.

When the security aspects are complied with, ventilation and/or drying processes are executed 210/212 if selected by the user.

After the possible ventilation and/or drying processes, the ozone cleaning may start. The device space comprises an inlet opening. Through the inlet opening, the device space receives 214 air from the exterior of the cabinet. Air may also be conveyed to the device space from the cleaning space. In the device space, ozone is generated 216 from the oxygen present in the air received from the exterior of the cabinet and/or from the cleaning space. The generated ozone mixed with air is conveyed 218 to the cleaning space via a passage connecting the device space and the cleaning space.

In the cleaning space, the ozone reacts with the impurities present in the objects to be cleaned. As the reaction product, oxygen and dead organic material are obtained. The dead, physically tangible organic material remains on the objects or falls to the floor of the cleaning space, for instance. The material may later on be removed from the objects by wiping with a cloth or by vacuum-cleaning, for instance.

The gaseous part of the reaction product including oxygen, ozone and air, is conveyed 220, via a second passage connecting the two spaces, to the device space. The ozone present in gas received in the device space needs to be neutralized before allowing the gas to exit the cabinet. In the neutralizer, the ozone is neutralized 210 to air, whereby it may be allowed to be conveyed to the surroundings of the cabinet, that is, to the room hosting the cabinet or to the outside air of the building including the cabinet.

In an embodiment, there is provided a way to determine if the objects to be cleaned are sufficiently clean from impurities. In this embodiment, the ozone concentration in the cleaning space is measured either continuously or discretely.

At first, the gas flows between the device space and the cleaning space are stopped. That is, the input of the ozone and air mixture from the device space to the cleaning space and the output of the gaseous reaction product from the cleaning space to the device space are stopped. The cleaning space is thus isolated from the surroundings such that no gas flow to/from the cleaning space is allowed to take place.

The ozone concentration in the cleaning space is measured 222 in connection with isolation of the cleaning space from the surroundings.

Subsequently, when the cleaning space has been isolated and as the ozone continues to react with the impurities in the objects to be cleaned, the ozone concentration in the cleaning space declines over time. Depending on the declining speed of the ozone concentration in the cleaning space, the following cleaning process may be controlled. For instance, if the ozone concentration declines more slowly than a predetermined threshold condition, it may be concluded that the objects to be cleaned are sufficiently clean from impurities and the process may continue to neutralization 224. However, if the ozone concentration in the cleaning space declines more quickly than a predetermined threshold condition, it may be concluded that the objects are not yet sufficiently clean and the actual cleaning process should be continued by returning to 214. The gas flows to and from the cleaning space may thus be resumed.

The threshold conditions for the ozone concentration declining speed may depend on the quantity or type of the material to be cleaned. Such threshold conditions may be predetermined conditions, or they may be calculated based on parameters given by the user.

In an embodiment, the ending of the cleaning process is monitored such that the use of the cabinet is safe. In an embodiment, the ozone generation in the device space is stopped, whereby also the input of the ozone to the cleaning space is stopped. Instead, only air may be input to the cleaning space. The output of gaseous products from the cleaning space may continue. As the output from the cleaning space continues and only air is input, the ozone concentration in the cleaning space declines rapidly. When the ozone concentration in the cleaning space is below a predetermined security threshold measured in 226, only then the security settings of the cabinet are released 228 and the door of the cabinet is allowed to open.

The invention and the embodiments are not limited to the examples shown above but may vary within the scope of attached claims.

## Claims

1. A cabinet for cleaning clothes, comprising:
a cleaning space (2) for containing the clothes to be cleaned;
means for generating (6) ozone from air;
means for bringing (27) the generated ozone into contact with the clothes in the cleaning space (2) for allowing the ozone to react with impurities in the clothes; and
means for neutralizing (11) the ozone included in the reaction product to oxygen;
a device space (3);
**characterized in that** the device space (3) composes an in-let opening (17) for receiving air from the exterior of the cabinet, and **in that** ozone is generated, during ozone cleaning, from the oxygen present in the air received from the exterior of the cabinet..

2. The cabinet according to claim 1, further comprising:
means for stopping the ozone generation (6) and neutralization (11) and closing the cleaning space (2) from flow of gas into and out of the cleaning space (2);
means for measuring the ozone concentration in the cleaning space (2); and
means for controlling the ozone generation (6) and/or the neutralization (11) based on the declining speed of the ozone concentration in the cleaning space (2).

3. The cabinet according to claim 2, further comprising:
means for resuming the cleaning process by restarting the ozone generation (6) and neutralization (11) if the declining speed of the ozone concentration exceeds a predetermined threshold condition; and
means for concluding the objects to be cleaned to be sufficiently clean if the declining speed of the ozone concentration in the cleaning space (2) undershoots a predetermined threshold condition.

4. The cabinet according to any preceding claim, further comprising:
means for delaying the ozone generation (6) for a predetermined time after starting a cleaning program; and
means for keeping a door (5) of the cleaning space (2) to be openable with a minor force during the time period when the ozone generation (6) is delayed.

5. The cabinet according any preceding claim, further comprising:
means for detecting motion within the cleaning space (2); and
means for preventing the ozone generation (6) when motion is detected within the cleaning space (2) before the door (5) of the cleaning space (2) is opened.

6. The cabinet according to any preceding claim, further comprising:
means for stopping the ozone generation (6);
means for neutralizing (11) ozone while the ozone generation (6) is stopped;
means for measuring the ozone concentration in the cleaning space (2) while the ozone generation (6) is stopped and the ozone neutralization (11) is in progress;
means for allowing the door (5) of the cleaning space (2) to be opened only if the ozone concentration in the cleaning space (2) undershoots a predetermined threshold value for the ozone concentration.

7. The cabinet according any preceding claim, wherein
the ozone generating (6) means is arranged outside the cleaning space whereby the ozone generated in the outside of the cleaning space (2) is conveyed to the interior of the cleaning space (2); and
the neutralizing means (11) is arranged outside the cleaning space (2) to convey the reaction product to the outside of the cleaning space (2) for neutralization.

8. The cabinet according to claim 7, wherein
the ozone generating means (6) and the neutralizing means (11) are arranged in a device space (3) separated by a wall (4) from the cleaning space (2), wherein the device space (3) comprises an inlet opening (17) for replacement air to be conveyed to the ozone generating (6) means, and the device space (3) comprises an outlet opening (15) for outputting oxygen output from the neutralizing (11) means.

9. The cabinet according to any preceding claim, further comprising:
means for blowing (28) the replacement air towards the ozone generating (6) means such as to provide an overpressure into the arrangement and prevent ozone from escaping the cleaning space (2) via the ozone generating means (6).

10. The cabinet according to any preceding claim, further comprising:
means for measuring humidity in the cleaning space (2), and wherein the ozone generating (6) means is configured to perform the ozone generation at a predetermined humidity level in the cleaning space (2).

11. The cabinet according to any preceding claim, wherein the neutralizing (11) means is configured to:
convey the reaction product through a cleaning chamber full of neutralizing matter.

## Patentansprüche

1. Ein Schrank zum Reinigen von Kleidung, der Folgendes aufweist:
einen Reinigungsraum (2) für die zu reinigende Kleidung;
Mittel zur Generierung (6) von Ozon aus Luft;
Mittel, um das generierte Ozon in Kontakt mit der Kleidung in dem Reinigungsraum (2) zu bringen (27), um das Ozon mit den Unreinheiten in der Kleidung reagieren zu lassen; und
Mittel zur Neutralisierung (11) des in dem Reaktionsprodukt enthaltenen Ozons zu Sauerstoff;
einen Geräteraum (3);
**dadurch gekennzeichnet, dass** der Geräteraum (3) eine Einlassöffnung (17) aufweist, um Luft von der Außenseite des Schranks zu empfangen, und dass Ozon generiert wird, während der Ozonreinigung, von dem in der Luft vorhandenen Sauerstoff, welche Luft von der Außenseite des Schranks erhalten wird.

2. Der Schrank gemäß dem Patentanspruch 1, der weiterhin Folgendes aufweist:
Mittel zum Unterbrechen der Ozongenerierung (6) und Neutralisierung (11) und Schließung des Reinigungsraums (2), so dass Gas nicht in den Reinigungsraum (2) hineinfließt oder aus ihm herausfließt;
Mittel zum Messen der Ozonkonzentration in dem Reinigungsraum (2); und
Mittel zur Kontrolle der Ozongenerierung (6) und/oder der Neutralisierung (11) auf der Basis der Abnahmegeschwindigkeit der Ozonkonzentration in dem Reinigungsraum (2).

3. Der Schrank gemäß dem Patentanspruch 2, der weiterhin Folgendes aufweist:
Mittel zum Wiederaufnehmen des Reinigungsprozesses, indem die Ozongenerierung (6) und die Neutralisation (11) wieder aufgenommen wird, falls die Abnahmegeschwindigkeit der Ozonkonzentration einen im Voraus bestimmten Stand überschreitet; und
Mittel zum Folgern, dass die zu reinigenden Objekte genügend rein sind, falls die Abnahmegeschwindigkeit der Ozonkonzentration in dem Reinigungsraum (2) einen im Voraus bestimmten Stand unterschreitet.

4. Der Schrank gemäß einem der vorhergehenden Patentansprüche, der weiterhin Folgendes aufweist:
Mittel zum Hinauszögern der Ozongenerierung (6) für eine im Voraus bestimmte Zeit nach dem Beginn des Reinigungsprogramms; und
Mittel, um das Öffnen der Tür (5) des Reinigungsraums (2) mit einer kleinen Kraft während derjenigen Zeitspanne zu erlauben, in welcher die Ozongenerierung (6) hinausgezögert wird.

5. Der Schrank gemäß einem der vorhergehenden Patentansprüche, der weiterhin Folgendes aufweist:
Mittel zum Wahrnehmen von Bewegung innerhalb des Reinigungsraums (2); und
Mittel zum Verhindern der Ozongenerierung (6), wenn Bewegung innerhalb des Reinigungsraums (2) wahrgenommen wird, bevor die Tür (5) des Reinigungsraums (2) geöffnet wird.

6. Der Schrank gemäß einem der vorhergehenden Patentansprüche, der weiterhin Folgendes aufweist:
Mittel zum Unterbrechen der Ozongenerierung (6);
Mittel zur Neutralisierung (11) von Ozon, während die Ozongenerierung (6) unterbrochen ist;
Mittel zum Messen der Ozonkonzentration in dem Reinigungsraum (2), während die Ozongenerierung unterbrochen ist und die Neutralisierung (11) von Ozon vor sich geht;
Mittel, um das Öffnen der Tür (5) des Reinigungsraums (2) nur zu erlauben, wenn die Ozonkonzentration in dem Reinigungsraum (2) einen im Voraus bestimmten Schwellenwert für die Ozonkonzentration unterschreitet.

7. Der Schrank gemäß einem der vorhergehenden Patentansprüche, wobei
das Mittel zur Ozongenerierung (6) außerhalb des Reinigungsraums angeordnet ist, wobei das außerhalb des Reinigungsraums (2) generierte Ozon in das Innere des Reinigungsraums (2) befördert wird; und
das Mittel zur Neutralisierung (11) außerhalb des Reinigungsraums (2) angeordnet ist, um das Reaktionsprodukt auf die Außenseite des Reinigungsraums (2) für die Neutralisierung zu befördern.

8. Der Schrank gemäß dem Patentanspruch 7, wobei
das Mittel zur Ozongenerierung (6) und das Mittel zur Neutralisierung (11) in einem, durch eine Wand (4) von dem Reinigungsraum (2) getrennten Geräteraum (3) angeordnet sind, wobei der Geräteraum (3) eine Einlassöffnung (17) aufweist, um die Ersatzluft in das Mittel zur Ozongenerierung (6) zu befördern, und der Geräteraum (3) eine Auslassöffnung (15) aufweist, um den Sauerstoffausstoß aus dem Mittel zur Neutralisierung (11) hinaus zu befördern.

9. Der Schrank gemäß einem der vorhergehenden Patentansprüche, welcher weiterhin Folgendes aufweist:
Mittel zum Blasen (28) von Ersatzluft in Richtung auf das Mittel zur Ozongenerierung (6), um einen Überdruck in der Anordnung zustande zu bringen und zu verhindern, dass Ozon aus dem Reinigungsraum (2) über die Mittel zur Ozongenerierung (6) ausströmt.

10. Der Schrank gemäß einem der vorhergehenden Patentansprüche, der weiterhin Folgendes aufweist:
Mittel zum Messen der Feuchtigkeit in dem Reinigungsraum (2), und wobei das Mittel zur Ozongenerierung (6) angeordnet ist, die Ozongenerierung bei einem im Voraus bestimmten Niveau der Feuchtigkeit in dem Reinigungsraum (2) durchzuführen.

11. Der Schrank gemäß einem der vorhergehenden Patentansprüche, wobei das Mittel zur Neutralisierung (11) angeordnet ist,
das Reaktionsprodukt durch eine, mit neutralisierendem Stoff gefüllte Reinigungskammer zu befördern.

## Revendications

1. Armoire pour le nettoyage de vêtements, comprenant :
un espace de nettoyage (2) pour contenir les vêtements devant être nettoyés ;
des moyens pour générer (6) de l'ozone à partir de l'air ;
des moyens pour amener (27) l'ozone généré en contact avec les vêtements dans l'espace de nettoyage (2) afin de permettre à l'ozone de réagir avec des impuretés dans les vêtements, et
des moyens pour neutraliser (11) l'ozone compris dans le produit de réaction en oxygène ;
un espace de dispositif (3) ;
**caractérisée en ce que** l'espace de dispositif (3) comprend une ouverture d'admission (17) permettant de recevoir l'air en provenance de l'extérieur de l'armoire, et **en ce que** l'ozone est généré, pendant le nettoyage à l'ozone, à partir de l'oxygène présent dans l'air reçu en provenance de l'extérieur de l'armoire.

2. Armoire selon la revendication 1, comprenant en outre :
des moyens pour arrêter la génération (6) et la neutralisation (11) d'ozone et pour fermer l'espace de nettoyage (2) à un écoulement de gaz dans et hors de l'espace de nettoyage (2) ;
des moyens pour mesurer la concentration en ozone dans l'espace de nettoyage (2) ; et
des moyens pour commander la génération (6) et/ou la neutralisation (11) d'ozone en se basant sur la vitesse de diminution de la concentration en ozone dans l'espace de nettoyage (2).

3. Armoire selon la revendication 2, comprenant en outre :
des moyens pour reprendre le processus de nettoyage en redémarrant la génération (6) et la neutralisation (11) d'ozone si la vitesse de diminution de la concentration en ozone dépasse une condition de seuil prédéterminée ; et
des moyens pour conclure que les objets à nettoyer sont assez propres si la vitesse de diminution de la concentration en ozone dans l'espace de nettoyage (2) n'atteint pas une condition de seuil prédéterminée.

4. Armoire selon l'une quelconque des revendications précédentes, comprenant en outre :
des moyens pour reporter la génération d'ozone (6) pendant une durée prédéterminée après le démarrage d'un programme de nettoyage ; et
des moyens permettant à une porte (5) de l'espace de nettoyage (2) de pouvoir continuer à s'ouvrir avec un minimum de force pendant la période où la génération d'ozone (6) est reportée.

5. Armoire selon l'une quelconque des revendications précédentes, comprenant en outre :
des moyens pour détecter un mouvement à l'intérieur de l'espace de nettoyage (2) ; et
des moyens pour empêcher la génération d'ozone (6) lorsqu'un mouvement est détecté à l'intérieur de l'espace de nettoyage (2) avant que la porte (5) de l'espace de nettoyage (2) soit ouverte.

6. Armoire selon l'une quelconque des revendications précédentes, comprenant en outre :
des moyens pour arrêter la génération d'ozone (6) ;
des moyens pour neutraliser (11) l'ozone pendant que la génération d'ozone (6) est arrêtée ;
des moyens pour mesurer la concentration en ozone dans l'espace de nettoyage (2) pendant que la génération d'ozone (6) est arrêtée et que la neutralisation d'ozone (11) est en cours ;
des moyens pour autoriser l'ouverture de la porte (5) de l'espace de nettoyage (2) que si la concentration en ozone dans l'espace de nettoyage (2) n'atteint pas une valeur seuil prédéterminée pour la concentration en ozone.

7. Armoire selon l'une quelconque des revendications précédentes, dans laquelle
les moyens de génération d'ozone (6) sont agencés à l'extérieur de l'espace de nettoyage, moyennant quoi l'ozone généré à l'extérieur de l'espace de nettoyage (2) est acheminé à l'intérieur de l'espace de nettoyage (2) ; et
les moyens de neutralisation (11) sont agencés à l'extérieur de l'espace de nettoyage (2) pour acheminer le produit de réaction vers l'extérieur de l'espace de nettoyage (2) pour neutralisation.

8. Armoire selon la revendication 7, dans laquelle
les moyens de génération d'ozone (6) et les moyens de neutralisation (11) sont agencés dans un espace de dispositif (3) séparé par une paroi (4) de l'espace de nettoyage (2), où l'espace de dispositif (3) comprend une ouverture d'admission (17) permettant de remplacer l'air devant être acheminé vers le moyen de génération d'ozone (6), et l'espace de dispositif (3) comprend une ouverture de refoulement (15) pour fournir en sortie de l'oxygène produit par le moyen de neutralisation (11).

9. Armoire selon l'une quelconque des revendications précédentes, comprenant en outre :
des moyens pour souffler (28) l'air de remplacement vers le moyen de génération d'ozone (6) de façon à fournir une surpression dans l'agencement et à empêcher l'ozone de s'échapper de l'espace de nettoyage (2) via les moyens de génération d'ozone (6).

10. Armoire selon l'une quelconque des revendications précédentes, comprenant en outre :
des moyens pour mesurer l'humidité dans l'espace de nettoyage (2), et où les moyens de génération d'ozone (6) sont configurés pour réaliser la génération d'ozone à un niveau d'humidité prédéterminé dans l'espace de nettoyage (2).

11. Armoire selon l'une quelconque des revendications précédentes, dans laquelle les moyen de neutralisation (11) sont configurés pour :
acheminer le produit de réaction dans une chambre de nettoyage pleine de matière neutralisante.
